# EUROPÄISCHE PATENTSCHRIFT

(11) **EP 0 502 509 B1**
(45) Veröffentlichungstag und Bekanntmachung des Hinweises auf die Patenterteilung: **11.09.1996**
(21) Anmeldenummer: 92103701.6
(22) Anmeldetag: 04.03.1992
(51) Int. Cl.: A61B 17/04, A61F 2/08, A61B 17/58, F16B 13/08

(54) **Knochendübel zur Fadenfixierung**
Bone dowel for fixing threads
Cheville à os pour fixer des fils

(30) Priorität: 04.03.1991 DE 4106823
(43) Veröffentlichungstag der Anmeldung: 09.09.1992
(73) Patentinhaber: LIEBSCHER KUNSTSTOFFTECHNIK, D-82166 Gräfelfing (DE); ARTHREX MEDIZINISCHE INSTRUMENTE GmbH, D-85757 Karlsfeld (DE)
(72) Erfinder: Metzler, Richard, W-8028 Taufkirchen (DE); Schmieding, Reinhold, W-8037 Olching (DE); Schmid, Peter M., W-8000 München 50 (DE)
(74) Vertreter: Köster, Hajo, Dr.

(56) Entgegenhaltungen:
- EP-A- 0 241 240
- EP-A- 0 340 159
- WO-A-89/01767
- WO-A-89/10096
- DE-A- 2 451 298
- FR-A- 2 264 214
- US-A- 1 808 318
- US-A- 3 089 377
- US-A- 3 954 345
- US-A- 4 738 255

## Beschreibung

Die Erfindung betrifft einen Knochendübel zur Fadenfixierung.

Bei chirurgischen Eingriffen besteht häufig die Notwendigkeit, ein Gewebe, beispielsweise Muskeln oder Faszien, an einem Knochen zu fixieren. Dazu kann man beispielsweise das Gewebe mit einer Schraube, einer Klammer oder einem Nagel an dem Knochen befestigen, so daß das Gewebe beispielsweise wieder an dem Knochen anwachsen kann. Bei einer derartigen Befestigung des Gewebes an einem Knochen bildet sich jedoch häufig aufgrund der von den Befestigungsmitteln ausgeübten lokalen hohen Drücke eine Gewebenekrose.

Aus der US-A-4 738 255 und der darauf basierenden EP-A-0 241 240 ist eine Fadenankervorrichtung bekannt, bei der der eigentliche, in einer Bohrung in einem Knochen zu plazierende Anker aus zwei separaten Teilen besteht, nämlich aus einem Dübelkörper bzw. einem geschlitzten Ring und aus einer Niete bzw. einem Spreizteil. Diese beiden Teile können mit Hilfe einer dort beschriebenen geeigneten Vorrichtung insbesondere beim Einsetzen zusammengehalten werden. Zudem kann die Niete bzw. der Spreizteil mit Hilfe dieser Vorrichtung nach dem Einsetzen in den Knochen in den geschlitzten Ring bzw. den Dübelkörper unter dessen Spreizung hineingezogen werden. Der in den Knochen eingesetzte Anker bzw. Dübel kann zur Fixierung eines Fadens dienen, mit dessen Hilfe Gewebe an dem Knochen befestigt etc. wird.

Aufgabe der vorliegenden Erfindung ist es, einen verbesserten Dübel bereitzustellen, der in ein in einem Knochen gebohrtes Loch eingesetzt und zur Fixierung eines Fadens dienen kann, wobei durch Ziehen an diesem Faden der Dübel am eingesetzten Ort fixiert werden kann.

Gelöst wird diese Aufgabe durch die Lehre des Anspruchs 1.

Der erfindungsgemäße Knochendübel besitzt einen in etwa zylindrischen Dübelkörper, der eine axiale durchgehende Längsbohrung sowie mindestens zwei sich bis zu einem Ende des Dübelkörpers erstreckende Längsschlitze aufweist. Die Längsschlitze erstrecken sich dabei nur über einen Teil der axialen Länge des Dübelkörpers und münden an einem Ende des Dübelkörpers bzw. sind dort "offen".

Zur Befestigung des erfindungsgemäßen Dübelkörpers in einem Knochen wird in letzteren ein Loch gebohrt, dessen Durchmesser so bemessen ist, daß der erfindungsgemäße Knochendübel bzw. der Dübelkörper paßgenau in dieses Loch eingesetzt werden kann. Der Dübelkörper des erfindungsgemäßen Knochendübels besitzt an demjenigen Ende, welches in das im Knochen ausgenommene Loch eingesetzt wird, einen in etwa stiftartigen Spreizteil. Der Dübelkörper und der Spreizteil besitzen dabei eine gemeinsame Längsachse. Mit anderen Worten, die Längsachse des Dübelkörpers und des Spreizteils fallen zusammen bzw. fluchten.

Der Spreizteil ist mit seinem zum Dübelkörper hin zeigenden Ende in dasjenige Ende des Dübelkörpers, an dem die Längsschlitze münden, in die Längsbohrung eingesetzt und dort fixiert. Mit anderen Worten, der Spreizteil ragt um einen geringen Betrag in die Längsbohrung des Dübelkörpers hinein.

Bei einer auf den Spreizteil in Richtung der Längsachse sowie zum Dübelkörper hin ausgeübten Kraft, die einen bestimmten Wert überschreitet, wird der Spreizteil in die Längsbohrung und somit in den Dübelkörper hineingezogen und spreizt dabei den Dübelkörper, so daß dieser in dem im Knochen gebohrten Loch festgeklemmt wird.

Diese Kraft wird vom Operateur durch einen Faden übertragen, der in das freie Ende des Dübelkörpers eingeführt ist, durch die Längsbohrung hindurch verläuft, seitlich aus einem Längsschlitz austritt, durch eine insbesondere am freien Ende des Spreizteils quer zur Längsachse angeordnete durchgehende Bohrung durchgeführt ist, zum zweiten Längsschlitz zurückgeführt ist, dort wieder in die Längsbohrung eintritt und aus dem freien Ende des Dübelkörpers wieder austritt. Durch gleichzeitiges Ziehen an den beiden am freien Ende des Dübelkörpers befindlichen und freiliegenden Fadenabschnitten wird der Faden gespannt und zieht bei Ausüben eines ausreichend großen Zuges den Spreizteil in die Längsbohrung des Dübelkörpers unter Spreizung des Dübelkörpers in diesem Bereich. Dabei muß der Operateur jedoch den Dübelkörper mit einem geeigneten Instrument niederhalten, damit der noch lose in das Loch im Knochen eingesetzte Knochdübel nicht insgesamt herausgezogen wird.

Die Querschnitt der Längsbohrung und der Querschnitt des stiftartigen Spreizteils können im Prinzip beliebiger Form, beipielsweise mehreckig, sein. Die Querschnitte der Längsbohrung sowie des Spreizteils sollten zweckmäßigerweise flächenkongruent sein.

Zur Erzielung einer ausreichenden Spreizung des Dübelkörpers nimmt der Querschnitt des Spreizteils beginnend von dem in den Dübelkörper eingesetzten Ende zum freien Ende hin kontinuierlich zu.

Nachdem der Spreizteil in den Dübelkörper hineingezogen worden ist, ist der Dübelkörper in dem im Knochen ausgenommenen Loch ortsfest fixiert. Mit den aus dem Dübel herausgeführten Fadenabschnitten kann Gewebe auf beliebige weise, beispielsweise durch Festbinden, Nähen, etc. am Knochen, in den der Dübel eingesetzt ist, fixiert werden.

Nach einer bevorzugten Ausführungsform besitzt die Längsbohrung im Dübelkörper einen kreisförmigen Querschnitt, der über die axiale Länge des Dübelkörpers konstant ist. Der Spreizteil ist ein Spreizkonus; somit ist der Querschnitt bzw. die Querschnittsfläche ein Kreis bzw. eine Kreisfläche, wobei der Radius dieses Kreises bzw. dieser Kreisfläche ausgehend von dem in den Dübelkörper eingesetzten Ende des Spreizkonus zum freien Ende des Spreizkonus kontinuierlich zunimmt. Auf diese Weise wird eine besonders große Spreizwirkung erzielt.

Der Spreizkonus sollte an seinem freien Ende einen Durchmesser aufweisen, der geringfügig kleiner ist als der Durchmesser des Dübelkörpers an dessen freiem Ende, da beidseitig vom Spreizkonus der Faden vorbeigeführt ist und in der Bohrung im Knochen einen gewissen Platz beansprucht. Der Durchmesser des Spreizkonus an seinem freien Ende sollte daher um einen Betrag gerin ger sein als der Durchmesser des Dübelkörpers an seinem freien Ende, wobei dieser Betrag in etwa dem doppelten Durchmesser des Fadens entspricht.

Nach einer weiterhin bevorzugten Ausführungsform ist der Innendurchmesser der Längsbohrung des Dübelkörpers an demjenigen Ende, in das der Spreizkonus eingesetzt ist, in etwa gleich groß wie der Außendurchmesser des Spreizkonus an seinem in die Längsbohrung eingesetzten Ende. Mit anderen Worten, die Außenmantelfläche des Spreizkonus liegt in etwa an der Innenmantelfläche der Innenbohrung an. Der Spreizkonus ist dabei nur so weit und somit nur einen gewissen Betrag in die Längsbohrung eingesetzt, daß seine axiale Ausrichtung und Fixierung sichergestellt ist.

Der aus dem Dübelkörper und dem Spreizkonus aufgebaute Knochendübel ist einstückig spritzgeformt. Der Übergang von der Außenmantelfläche des Spreizkonus in dem Bereich, in dem der Spreizkonus in den Dübelkörper eingesetzt ist, auf die Innenmantelfläche der Längsbohrung des Dübelkörpers stellt dabei eine Sollbruchstelle dar. Diese bricht, wenn der Spreizkonus mit Hilfe des Fadens unter Anwendung einer ausreichend starken Kraft in den Dübelkörper hineingezogen wird.

Dabei kann die Außenmantelfläche des Spreizkonus im Überlappungsbereich mit der Innenmantelfläche der Längsbohrung zusammenfallen bzw. daran anliegen. Jedoch kann an diesem Übergang auch eine Art Ring zwischen der Innenmantelfläche der Längsbohrung und der Außenmantelfläche des Spreizkonus vorhanden sein. Dieser Ring ist sowohl mit dem Dübelkörper als auch mit dem Spreizkonus einstückig geformt.

Der erfindungsgemäße Knochendübel kann aus jedem körperverträglichem Material gefertigt sein, beispielsweise aus Polycarbonat. Vorzugsweise ist der erfindungsgemäße Knochendübel aus einem vom Körper resorbierbaren Material, beispielsweise Polylactat bzw. Polylactid oder PDS gefertigt. Auch der Faden ist vorzugsweise aus einem resorbierbaren Material gefertigt. Derartige Fäden sind allgemein bekannt. Sind der Knochendübel und/oder der Faden aus einem resorbierbaren Material gefertigt, dann lösen sich diese nach einer gewissen Zeit und nach Anwachsen des damit befestigten Gewebes am Knochen auf, so daß die Befestigungsmittel zur Fixierung des Gewebematerials nach einer gewissen Zeit nicht mehr vorhanden sind. Im Körper befinden sich dann keine zur Fixierung des Gewebes eingesetzten Fremdstoffe mehr.

Weitere bevorzugte Ausführungsformen sind in den Unteransprüchen 6 bis 10 beschrieben.

Der erfindungsgemäße Knochedübel wird anhand der folgenden, skizzenhaften, eine bevorzugte Ausführungsform darstellenden Figuren näher erläutert. Von diesen Figuren zeigen:
- Fig. 1: eine Seitenansicht, teilweise geschnitten, eines erfindungsgemäßen Knochendübels;
- Fig. 2: einen Schnitt entlang der Linie A-A der Fig. 1;
- Fig. 3: einen Schnitt entlang der Linie B-B der Fig. 1; und
- Fig. 4: eine Vergrößerung des Ausschnittes C.

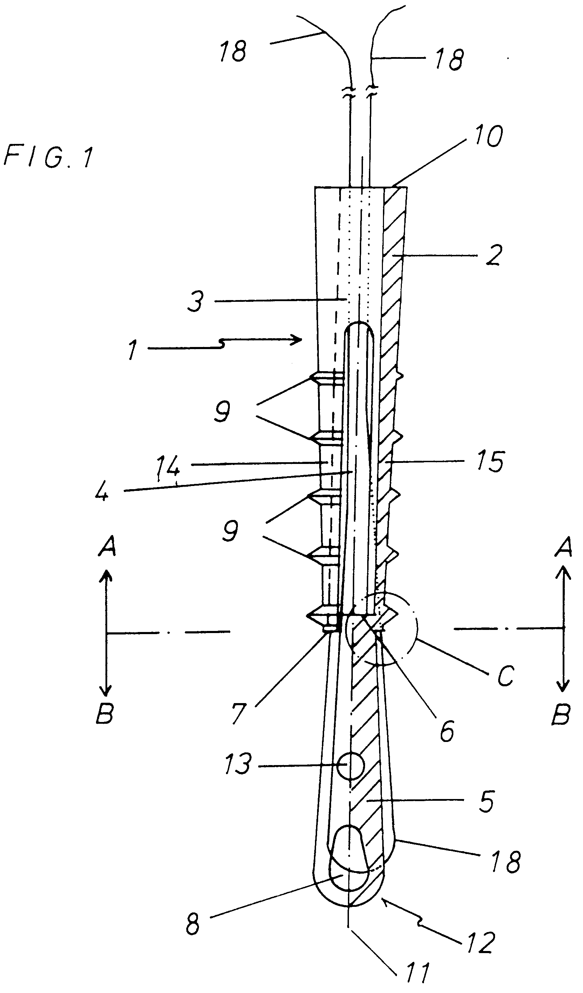

Der in der Fig. 1 gezeigte Knochendübel 1 besteht aus einem in etwa zylindrischen Dübelkörper 2 und einem Spreizkonus 5. Der Dübelkörper 2 besitzt einen kreisförmigen Querschnitt, dessen Radius ausgehend von seinem freien Ende 10 zum Ende 7 kontinuierlich abnimmt. Mit anderen Worten, der in etwa zylindrische Dübelkörper verjüngt sich zum Spreizkonus 5 hin.

Der Dübelkörper 2 besitzt eine zentrale durchgehende Längsbohrung 3 mit einem über die axiale Länge gleichbleibenden kreisförmigen Querschnitt.

Ausgehend vom Ende 7 des Dübelkörpers 2 erstrecken sich zwei diametral gegenüberliegende Längsschlitze 4 bis über die Mitte des Dübelkörpers hinaus, so daß zwei einander gegenüberliegende Zungen 14,15 gebildet werden. Auf diesen Zungen 14,15 sind mehrere, radial um den Dübelkörper 2 umlaufende Rippen 9 angebracht, deren Außendurchmesser in etwa dem Durchmesser des Dübelkörpers an seinem freien Ende 10 entspricht. Da sich der Dübelkörper 2 zu seinem Ende 7 hin konisch verjüngt, bedeutet dies für die Rippen 9, daß der Innendurchmesser dieser radial und somit kreisartig umlaufenden Rippen ausgehend vom Ende 7 abnimmt. Mit anderen Worten, die dem Ende 7 am nächsten liegende Rippe 9 ist am "stärksten ausgebildet", während die "Stärke" der Rippen zum Ende 10 hin abnimmt.

In das Ende 7 des Dübelkörpers 2 bzw. der Längsbohrung 3 ist das Ende 6 des Spreizkonus eingesetzt. Der Spreizkonus ragt somit einen gewissen Betrag in die Längsbohrung 4 hinein.

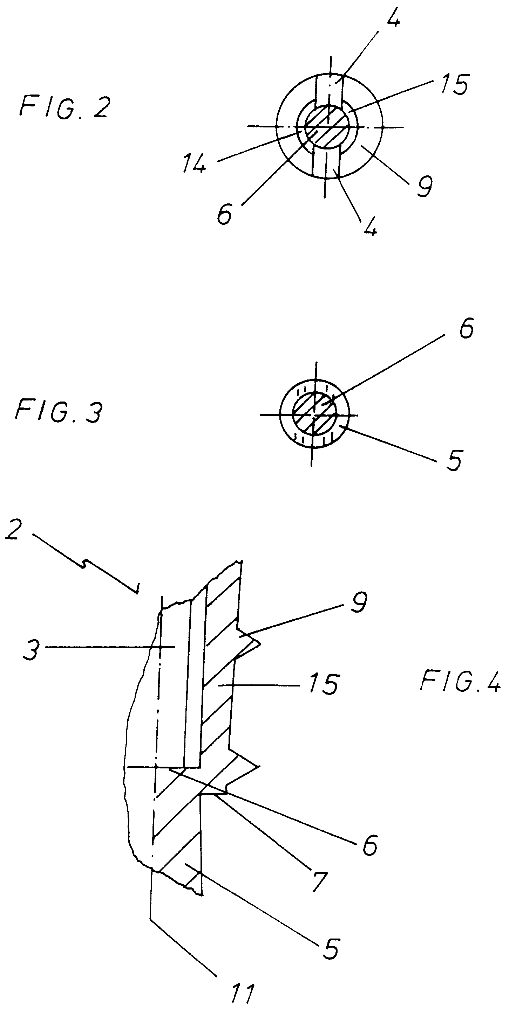

Der Dübelkörper 2 und der Spreizkörper 5 sind einstückig spritzgeformt. Wie man insbesondere aus der Fig. 4 ersieht, geht der Spreizkonus in dem Bereich, in dem sich Dübelkörper und Spreizkonus überlappen (Überlappungsbereich), ineinander über. Die Außenmantelfläche des Spreizkonus und die Innenmantelfläche der Längsbohrung 3 des Dübelkörpers 2 "fallen somit zusammen".

Um den erfindungsgemäßen Knochendübel 1 zum Einsatz zu bringen, wird dieser in ein zuvor in einem Knochen ausgenommenen Loch bzw. einer entsprechenden Bohrung min dem Spreizkonus 5 voran in dieses Loch eingeführt. Das Loch im Knochen sollte so tief sein, daß der erfindungsgemäße Knochendübel vollständig darin aufgenommen wird.

In den erfindungsgemäßen Knochendübel ist zuvor ein Faden 18 eingeführt worden, dessen Verlauf nachstehend beschrieben ist. Dieser Faden 18 wird am freien Ende 10 in die Längsbohrung 3 eingeführt, verläuft dann von dort bis zu einem Längsschlitz 4, tritt dort seitlich parallel versetzt zur Längsachse 11 aus, wird seitlich parallel zum Spreizkonus 5 bis zu einer Bohrung 8 geführt, tritt durch diese hindurch, wird dann seitlich vom Spreizkonus 5 zurück zu demjenigen Längsschlitz 4 geführt, der dem zuerst genannten Längsschlitz 4 diametral gegenüberliegt, tritt dort wieder in die Längsbohrung 3 ein und wird am freien Ende 10 herausgeführt. Durch gleichzeitiges Ziehen an den aus dem freien Ende 12 des Dübelkörpers 2 austretenden Fadenabschnitten wird der Faden 18 gespannt und übt dann auf den Spreizkonus 5 eine Kraft in Richtung der Längsachse 11 sowie in Richtung des Dübelkörpers 2 aus. Überschreitet diese Kraft einen bestimmten Wert, dann bricht die Verbindung vom Spreizkonus 5 zum Dübelkörper 2 bzw. zu den Zungen 14,15 und der Spreizkonus 5 wird unter Auseinanderspreizen der Zungen 14 und 15 und somit unter Fixierung des Dübels in seinem Loch im Knochen in die Längsbohrung 3 des Dübelkörpers 2 hineingezogen. Dabei muß der Dübelkörper natürlich gleichzeitig in das Loch gedrückt gehalten werden, damit der Dübel nicht insgesamt herausgezogen wird.

Der Übergang vom Spreizkonus 5 zu den Zungen 14,15 des Dübelkörpers 2 stellt somit eine Sollbruchstelle dar. Die Kraft, die ausgeübt werden muß, damit an dieser Sollbruchstelle der gewünschte Bruch erfolgen kann, wird unter anderem durch die Länge festgelegt, welche der Spreizkonus 5 in die Längsbohrung 3 hineinragt. Je größer der Überlappungsbereich somit ist, desto größer ist auch die aufzuwendende Kraft, um das Material an dieser Stelle brechen zu lassen.

Die axiale Länge des Spreizkonus entspricht in etwa der axialen Länge der Längsschlitze 4.

Die Bohrung 8 besitzt einen oval-birnenförmigen Querschnitt bzw. eine oval-birnenförmige Form, wobei die längste Achse dieser Birnenform mit der Längsachse 11 des Knochendübels zusammenfällt. Die Wandung der Bohrung 8 ist in dem zum Ende 6 hin zeigenden Bereich (oberer Abschnitt in der Fig. 1; "Stengelabschnitt der Birne") zum freien Ende 12 des Spreizkonus 5 gewölbt, damit der Faden 18, ohne festgeklemmt zu werden, auch noch nach Einziehen des Spreizkonus 5 in die Längsbohrung 3 des Dübelkörpers 2 bewegt bzw. gezogen werden kann. Da der Wandungsbereich abgerundet ist, wird der Faden 18 auch nicht an irgendwelchen Kanten etc. festgeklemmt.

Der Spreizkonus 5 weist außerdem noch eine zweite durchgehende Bohrung 13 auf, die zwischen der Bohrung 8 und dem Ende 6 des Spreizkonus 5 senkrecht zur Längsachse 11angebracht ist. Diese zweite durchgehende Bohrung 13 ist streng zylindrisch und besitzt somit auch am Übergang auf die Außenmantelfläche des Spreizkonus 5 eine Kante. Wird der Faden 18 durch diese zweite Bohrung 13 geführt (und somit nicht durch die Bohrung 8, wie das in der Fig. 1 gezeigt ist), dann wird der Faden 18 an der genannten Kante der zweiten Bohrung 13 quasi senkrecht abgeknickt und kann nicht mehr durchgezogen werden. Je nachdem, ob man einen durchziehbaren Faden oder einen nicht-durchziehbaren Faden im Knochen anzubringen wünscht, führt man den Faden 18 durch die zweite Bohrung 13 oder durch die erste Bohrung 8. Die zweite Bohrung 13 sollte dabei einen Durchmesser besitzen, der in etwa dem Durchmesser des Fadens entspricht. Der Durchmesser der Bohrung 8 sollte jedoch größer sein als der Durchmesser des Fadens 18.

Wie man insbesondere der Fig. 3 entnehmen kann, verjüngt sich der Spreizkonus 5 zu seinem freien Ende 6 hin, an dem er in die Längsbohrung 3 des Dübelkörpers 2 eingesetzt ist.

Aus dem in der Fig. 2 gezeigten Schnitt A-A wird besonders deutlich, daß die Längsschlitze 4 einander diametral gegenüberliegen und zwischen zwei Zungen 14,15 verlaufen sowie am Ende 7 des Dübelkörpers 2 "ins Freie" münden.

Die Mitte bzw. die Mittellinien der Längsschlitze 4 und die Längsachsen der Bohrungen 8 und 13 liegen in einer Ebene, die in der Fig. 1 senkrecht zur Papierebene verläuft. In dieser Ebene befindet sich auch die Längsachse 11. Durch diese Anordnung wird gewährleistet. daß der Spreizkonus 5 völlig oder sogar noch tiefer ind den Dübelkörper hineingezogen werden kann, ohne daß die Zungen 14,15 die Bohrungen 8,13 abdecken und den Faden 18 dabei einklemmen. Daher entspricht auch die Breite der Längsschlitze 4 in etwa dem Durchmesser der Bohrungen 8,13.

Der erfindungsgemäße Knochendübel ist aus einem resorbierbaren Material, beispielsweise Polylactat, spritzgeformt.

## Patentansprüche

1. Knochendübel zur Fadenfixierung mit einem in etwa zylindrischen Dübelkörper (2),
der eine axiale durchgehende Längsbohrung (3) besitzt und der mindestens zwei sich bis zu einem Ende (7) erstreckende Längsschlitze (4) aufweist,
und mit einem in etwa stiftartigen Spreizteil (5),
der eine mit dem Dübelkörper (2) gemeinsame Längsachse (11) besitzt,
der mindestens eine in etwa senkrecht zur Längsachse (11) verlaufende durchgehende Bohrung (8) aufweist,
der mit dem Dübelkörper (2) einstückig spritzgeformt ist und der mit seinem zum Dübelkörper (2) zeigenden Ende (6) an dem Ende (7) des Dübelkörpers (2), an dem die Längsschlitze (4) münden, in die Längsbohrung (3) derart eingesetzt und dort fixiert ist, daß der Übergang von der Außenmantelfläche des Spreizteils (5) in dem Bereich, in dem dieser in den Dübelkörper (2) eingesetzt ist, auf die Innenmantelfläche der Längsbohrung (4) des Dübelkörpers (2) eine Sollbruchstelle darstellt, die bei Ausüben einer in Richtung der Längsachse (11) sowie zum Dübelkörper (2) gerichteten, einen vorbestimmten Wert überschreitenden Kraft bricht, so daß der Spreizteil (5) aus seiner fixierten Lage bewegt und in den Dübelkörper (2) unter dessen Spreizung hineingezogen wird.

2. Knochendübel nach Anspruch 1,
dadurch **gekennzeichnet**,
daß die Längsbohrung (3) einen kreisförmigen Querschnitt besitzt, der über die axiale Länge des Dübelkörpers (2) konstant ist, und daß der Spreizteil ein Spreizkonus (5) ist.

3. Knochendübel nach Anspruch 2,
dadurch **gekennzeichnet**,
daß der Innendurchmesser der Längsbohrung (3) am Ende (7) des Dübelkörpers (2) in etwa gleich groß ist wie der Außendurchmesser des Spreizkonus (5) an seinem in die Längsbohrung eingesetzten Ende (6).

4. Knochendübel nach einem der vorhergehenden Ansprüche,
dadurch **gekennzeichnet**,
daß er aus einem resorbierbaren Material, insbesondere Polylactat, gefertigt ist

5. Knochendübel nach einem der vorhergehenden Ansprüche,
dadurch **gekennzeichnet**,
daß der Außendurchmesser des Dübelkörpers (2) von seinem freien Ende (10) zu seinem gegenüberliegenden Ende (7) hin kontinuierlich abnimmt.

6. Knochendübel nach Anspruch 5,
**gekennzeichnet** durch
mehrere axial beabstandete, im Bereich der Längsschlitze (4) außen um den Dübelkörper (2) radial umlaufende Rippen (9), deren jeweiliger Außendurchmesser in etwa dem Außendurchmesser des Dübelkörpers (2) an seinem freien Ende (10) entspricht.

7. Knochendübel nach einem der vorhergehenden Ansprüche,
dadurch **gekennzeichnet**,
daß die senkrecht zur Längsachse verlaufende Bohrung (8) einen oval-birnenförmigen Querschnitt hat, dessen längste Achse mit der Längsachse (11) des Knochendübels (1) zusammenfällt, daß die Bohrung (8) am freien Ende (12) des Spreizteiles bzw. des Spreizkonus (5) angebracht ist und daß die zum Ende (6) hin liegende Wand der Bohrung (8) zum freien Ende (12) hin gewölbt ist.

8. Knochendübel nach einem der vorhergehenden Ansprüche,
**gekennzeichnet** durch
eine weitere quer zur Längsachse (11) verlaufende durchgehende Bohrung (13), die einen konstanten kreisförmigen Durchmesser besitzt und zwischen der Bohrung (8) und dem zum Dübelkörper (2) zeigenden Ende (6) angeordnet ist.

9. Knochendübel nach Anspruch 8,
dadurch **gekennzeichnet**,
daß die Längsschlitze (4) und beide Bohrungen (8,13) in einer Ebene liegen, in der auch die Längsachse (11) liegt, und daß die axiale Länge der Längsschlitze (4) in etwa der axialen Länge des Spreizkonus (5) entspricht.

## Claims

1. Bone dowel for anchoring a suture, comprising an approximately cylindrical dowel shank (2) having a continuous axial longitudinal borehole (3) and at least two longitudinal slots (4) extending to one end (7) and furthermore comprising an approximately pin-shaped expansion part (5) which
has a longitudinal axis (11) coincident with that one of the dowel shank (2),
includes at least one aperture (8) extending approximately perpendicular to the longitudinal axis (11),
is integrally molded with the dowel shank (2) and is inserted and fixed with its end (6), which is directed to the dowel shank (2) at that end (7) of the dowel shank (2) to which the longitudinal slots (4) are open, into the longitudinal borehole (3) in such a manner that the transition region of the outer surface of the expansion part (5) in that zone in which the latter is inserted into the dowel shank (2) to the inner surface of the longitudinal borehole (4) of the dowel shank (2) represents a predetermined breaking point which breaks when a force directed towards the direction of the longitudinal axis (11) as well as of the dowel shank (2) exceeds a predetermined value so that the expansion part (5) is moved away from its fixed position and is pulled into the dowel shank (2), thereby causing the dowel shank to expand.

2. Bone dowel according to claim 1,
**characterized in**
that the longitudinal borehole (3) has a circular cross section remaining constant along the axial lenght of the dowel shank (2) and that the expansion part is an expansion cone (5).

3. Bone dowel according to claim 2,
**characterized in**
that the inside diameter of the longitudinal borehole (3) at the end (7) of the dowel shank (2) is approximately equal to the outside diameter of the expansion cone (5) at its end (6) which is inserted into the longitudinal borehole.

4. Bone dowel according to one of the preceeding claims,
**characterized in**
that it is made of a resorbable material, in particular polylactate.

5. Bone dowel according to one of the preceeding claims,
**characterized in**
that the outer diameter of the dowel shank (2) continuously decreases from its free end (10) to its opposite end (7).

6. Bone dowel according to claim 5,
**characterized by**
a plurality of axially spaced ribs (9) located on a portion outside of the dowel shank (2) which includes the longitudinal slots (4) whereby the outside diameter of the ribs approximately equals the outside diameter of the dowel shank (2) at its free end (10).

7. Bone dowel according to one of the preceeding claims
**characterized in**
that the aperture (8) extending perpendicular to the longitudinal axis has an oval or pear shape of which a long axis coincides with the longitudinal axis (11) of the bone dowel (1), that the aperture (8) is located at the free end (12) of the expansion part or expansion cone (5), respectively, and that a wall of the aperture (8) nearest the end (6) is arcuate to the free end (12).

8. Bone dowel according to one of the preceeding claims,
**characterized by**
a further aperture (13) extending transverse to the longitudinal axis (11) and having a constant, circular cross-section and being located between the aperture (8) and the end (6) directed towards the dowel shank (2).

9. Bone dowel according to claim 8,
**characterized in**
that the longitudinal slots (4) and both apertures (8, 13) are located in a plane in which the longitudinal axis (11) is also located and that the axial lenght of the longitudinal slots (4) corresponds approximately to the axial lenght of the expansion cone (5).

## Revendications

1. Cheville à os pour la fixation de fils, comportant un corps approximativement cylindrique (2),
qui possède un perçage longitudinal axial traversant et comporte au moins deux fentes longitudinales (4) qui s'étendent jusqu'à une extrémité (7), et
comportant une pièce d'écartement (5) approximativement en forme de tige, qui possède un axe longitudinal (11) commun avec le corps (2) de la cheville, et
qui possède au moins un perçage traversant (8) qui s'étend approximativement perpendiculairement à l'axe longitudinal (11),
qui est formé par moulage par injection d'un seul tenant avec le corps (2) de la cheville et qui est inséré par son extrémité (6) dirigée vers le corps (2) de la cheville, dans le perçage longitudinal (3) de l'extrémité (7) du corps (2) de la cheville, dans laquelle débouchent les fentes longitudinales (4), et y est fixé de telle sorte que la jonction de la surface enveloppe extérieure de la pièce d'écartement (5), avec la surface enveloppe intérieure du perçage longitudinal (4) du corps (2) de la cheville dans la zone, dans laquelle la pièce d'écartement est insérée dans le corps (2) de la cheville, représente une zone de rupture de consigne, qui se rompt lors de l'application d'une force dirigée dans la direction de l'axe longitudinal (11) ainsi qu'en direction du corps (2) de la cheville et dépasse une valeur prédéterminée, de sorte que la pièce d'écartement (5) est déplacée à partir de sa position fixée et est introduite dans le corps (2) de la cheville, sous l'effet de l'écartement de ce corps.

2. Cheville à os selon la revendication 1, caractérisée en ce que le perçage longitudinal (3) possède une section transversale circulaire, qui est constante sur la longueur axiale du corps (2) de la cheville et que la pièce d'écartement est un cône d'écartement (5).

3. Cheville à os selon la revendication 2, caractérisée en ce que le diamètre intérieur du perçage longitudinal (3) dans l'extrémité (7) du corps (2) de la cheville est approximativement égal au diamètre extérieur du cône d'écartement (5) au niveau de son extrémité (6) insérée dans le perçage longitudinal.

4. Cheville à os selon l'une des revendications précédentes, caractérisée en ce qu'elle est réalisée en un matériau résorbable, notamment en polylactate.

5. Cheville à os selon l'une des revendications précédentes, caractérisée en ce que le diamètre extérieur du corps (2) de la cheville diminue continûment depuis son extrémité libre (10) en direction de son extrémité opposée (7).

6. Cheville à os selon la revendication 5, caractérisée par plusieurs nervures radiales circonférentielles (9) glissantes axialement, qui s'étendent extérieurement autour du corps (2) de la cheville et dont le diamètre extérieur effectif correspond approximativement au diamètre extérieur du corps (2) de la cheville au niveau de son extrémité libre (10).

7. Cheville à os selon l'une des revendications précédentes, caractérisée en ce que le perçage (8), qui s'étend perpendiculairement à l'axe longitudinal, possède une section transversale piriforme ovale, dont le grand axe coïncide avec l'axe longitudinal (11) de la cheville à os (1) de sorte que le perçage (8) est monté sur l'extrémité libre (12) de la pièce d'écartement ou du cône d'écartement (5), et que la paroi du perçage (8), dirigée vers l'extrémité (6), est cintrée en direction de l'extrémité libre (12).

8. Cheville à os selon l'une des revendications précédentes, caractérisée par un autre perçage traversant (13) qui s'étend transversalement par rapport à l'axe longitudinal (11), a une forme de cercle de diamètre constant et est disposé entre le perçage (8) et l'extrémité (6) tournée vers le corps (2) de la cheville.

9. Cheville à os selon la revendication 8, caractérisée en ce que les fentes longitudinales (4) et les deux perçages (8,13) sont situés dans un plan, dans lequel est également situé l'axe longitudinal (11), et que la longueur axiale des fentes longitudinales (4) correspond approximativement à la longueur axiale du cône d'écartement (5).
